# EUROPEAN PATENT APPLICATION

(11) **EP 4 722 343 A1**
(43) Date of publication of application: **08.04.2026**
(21) Application number: 24813101.3
(22) Date of filing: 29.05.2024
(51) Int. Cl.: C12N 5/071, C12N 5/10, C12Q 1/02

(54) **METHOD FOR PRODUCING SUPRACHIASMATIC NUCLEUS ORGANOID**

(30) Priority: 29.05.2023 JP 2023087986
(71) Applicant: Kansai Medical University Educational Corporation, City of Hirakata, Osaka 573-1010 (JP)
(72) Inventor: TAMIYA, Hiroyuki, Kyoto-shi, Kyoto 602-8566 (JP); EIRAKU, Mototsugu, Kyoto-shi, Kyoto 606-8501 (JP)
(74) Representative: Winter, Brandl - Partnerschaft mbB
(86) International application number: PCT/JP2024/019660
(87) International publication number: WO 2024/248023

(57) **Abstract**

The present invention aims to provide a method for producing a suprachiasmatic nucleus organoid. Provided is a method for producing a suprachiasmatic nucleus organoid, including: (1) a step of culturing pluripotent stem cells in an intermediate medium between an undifferentiated maintenance medium and a neural differentiation medium; (2) a step of culturing cells obtained in step (1) in the neural differentiation medium; and (3) a step of performing an operation for cell mass formation prior to the start of step (2), wherein (A) the number of raw material cells seeded to form one cell mass is 6000 or greater, and (B) when the start of step (2) is defined as Day 0, the medium contains a sonic hedgehog signaling pathway agonist prior to Day 3.

## Description

### Technical Field

The present invention relates to, for example, a method for producing a suprachiasmatic nucleus organoid.

### Background Art

Modern society is facing increasing problems associated with the disruption of circadian rhythms, along with greater lifestyle diversity. For example, night shifts, shift changes, long-distance travel, etc. can cause a mismatch between the sleep-wake rhythm and the external light cycle, resulting in sleep disorders due to the disruption of circadian rhythms. Such sleep disorders are risk factors of various diseases, including lifestyle-related diseases, cancer, and mental illnesses.

In mammals, the suprachiasmatic nucleus is the center of circadian rhythms. Thus, regulating the circadian rhythm in the suprachiasmatic nucleus is expected to improve sleep disorders and reduce the risk of the above-mentioned diseases. However, the mechanism to control the circadian rhythm in the suprachiasmatic nucleus remains largely unknown.

### Citation List

### Non Patent Literature

NPL 1: Proc Natl Acad Sci USA. 2008 Aug 19; 105(33): 11796-801
NPL 2: Nature. 2011 Nov 9; 480(7375): 57-62

### Summary of Invention

### Technical Problem

The present inventors focused on the possibility that suprachiasmatic nucleus organoids, if obtained, would enable further elucidation of the mechanism to control circadian rhythms and the development of new drugs to control circadian rhythm disorders.

NPL 1 reports a method for inducing hypothalamic neurons from mouse ES cells. However, this method does not involve suprachiasmatic nucleus induction. NPL 2 reports a method for inducing pituitary gland by inducing hypothalamus and oral epithelium simultaneously. However, this method does not involve suprachiasmatic nucleus induction.

The present invention aims to provide a method for producing a suprachiasmatic nucleus organoid.

### Solution to Problem

As a result of extensive study, the present inventors found that the above aim can be achieved by a method for producing a suprachiasmatic nucleus organoid, comprising:
(1) a step of culturing pluripotent stem cells in an intermediate medium between an undifferentiated maintenance medium and a neural differentiation medium;
(2) a step of culturing cells obtained in step (1) in the neural differentiation medium; and
(3) a step of performing an operation for cell mass formation prior to the start of step (2),
wherein
(A) the number of raw material cells seeded to form one cell mass is 6000 or greater, and (B) when the start of step (2) is defined as Day 0, the medium contains a sonic hedgehog signaling pathway agonist prior to Day 3.

As a result of further study based on the above finding, the present inventors completed the present invention. Specifically, the present invention encompasses the following embodiments.

Item 1. A method for producing a suprachiasmatic nucleus organoid, comprising:
(1) a step of culturing pluripotent stem cells in an intermediate medium between an undifferentiated maintenance medium and a neural differentiation medium;
(2) a step of culturing cells obtained in step (1) in the neural differentiation medium; and
(3) a step of performing an operation for cell mass formation prior to the start of step (2),
wherein
(A) the number of raw material cells seeded to form one cell mass is 6000 or greater, and
(B) when the start of step (2) is defined as Day 0, the medium contains a sonic hedgehog signaling pathway agonist prior to Day 3.

Item 2. The production method according to Item 1, wherein the intermediate medium in step (1) is a medium in which the concentration of an undifferentiated maintenance factor is lower than that in the undifferentiated maintenance medium.

Item 3. The production method according to Item 2, wherein the undifferentiated maintenance factor comprises an FGF-MAPK signal inhibitor and/or a Wnt signal activator.

Item 4. The production method according to any one of Items 1 to 3, wherein the number of raw material cells in step (3) is 9000 or greater.

Item 5. The production method according to any one of Items 1 to 4, wherein the operation in step (3) comprises seeding cells into culture wells and culturing the cells in suspension.

Item 6. The production method according to any one of Items 1 to 5, wherein culturing in the medium containing the sonic hedgehog signaling pathway agonist continues until a day between Day 4 and Day 15.

Item 7. The production method according to any one of Items 1 to 6, wherein culturing in step (2) continues until a day between Day 4 and Day 15.

Item 8. The production method according to any one of Items 1 to 7, wherein the concentration of the sonic hedgehog signaling pathway agonist in the medium is 0.5 µM or more.

Item 9. The production method according to any one of Items 1 to 8,
wherein the intermediate medium in step (1) is a medium in which the concentration of an undifferentiated maintenance factor is lower than that in the undifferentiated maintenance medium,
the undifferentiated maintenance factor comprises an FGF-MAPK signal inhibitor and/or a Wnt signal activator,
the number of the raw material cells in step (3) is 9000 or greater, the operation in step (3) comprises seeding cells into culture wells and culturing the cells in suspension,
culturing in the medium containing the sonic hedgehog signaling pathway agonist continues until a day between Day 4 and Day 15,
culturing in step (2) continues until a day between Day 4 and Day 15, and
the concentration of the sonic hedgehog signaling pathway agonist in the medium is 0.5 µM or more.

Item 10. The production method according to any one of Items 1 to 9, further comprising (3) a step of culturing cell masses obtained in step (2) in the neural differentiation medium under high oxygen conditions.

Item 11. A suprachiasmatic nucleus organoid, wherein the proportion of suprachiasmatic nucleus cells is 5% or more.

Item 12. The suprachiasmatic nucleus organoid according to Item 11, wherein the proportion of suprachiasmatic nucleus cells is 10% or more.

Item 13. A suprachiasmatic nucleus organoid obtainable by the production method according to any one of Items 1 to 10.

Item 14. A method for screening for a circadian rhythm regulator, comprising:
(X) a step of contacting the suprachiasmatic nucleus organoid according to any one of Items 11 to 13 with a test substance; and
(Y) a step of evaluating the circadian rhythm in the suprachiasmatic nucleus organoid.

Item 15. A circadian rhythm regulator having an action to change the circadian rhythm in the suprachiasmatic nucleus organoid according to Item 11 or 12.

### Advantageous Effects of Invention

The present invention can provide a method for producing a suprachiasmatic nucleus organoid, a suprachiasmatic nucleus organoid, a method for screening for a circadian rhythm regulator, and the like.

### Brief Description of Drawings

Fig. 1 shows the results from immunostaining of organoids obtained in Test Example 1-1 with terminal differentiation markers (Six6, VIP, and AVP) of the suprachiasmatic nucleus (Test Example 1-2).
Fig. 2 shows the results from analysis of clock gene expression in the organoids obtained in Test Example 1-1 (Test Example 1-4).
Fig. 3 shows an actogram obtained by measuring movements of SCN-disrupted mice before and after transplantation with the organoids obtained in Test Example 1-1 (Test Example 1-5).
Fig. 4 shows a chi-squared periodogram obtained by measuring movements of SCN-disrupted mice before and after transplantation with the organoids obtained in Test Example 1-1 (Test Example 1-5).
Fig. 5 shows brightfield images (Bright), fluorescence images (showing Six3 expression), and autofluorescence images (Auto) of cell masses at Day 13 (Test Example 2).
Fig. 6 shows brightfield image (Bright), fluorescence images (showing Six3 expression), and autofluorescence images (Auto) of cell masses at Day 48 (Test Example 3).
Fig. 7 shows brightfield images of cell masses at Day 13 (Test Example 4).

### Description of Embodiments

In the specification, the terms "include," "contain," and variations thereof express concepts that encompass all of "comprise," "consist essentially of," and "consist of."

### 1. Method for producing suprachiasmatic nucleus organoid

In one embodiment, the present invention relates to a method for producing a suprachiasmatic nucleus organoid, comprising: (1) a step of culturing pluripotent stem cells in an intermediate medium between an undifferentiated maintenance medium and a neural differentiation medium; (2) a step of culturing cells obtained in step (1) in the neural differentiation medium; and (3) a step of performing an operation for cell mass formation prior to the start of step (2), wherein (A) the number of raw material cells seeded to form one cell mass is 6000 or greater, and (B) when the start of step (2) is defined as Day 0, the medium contains a sonic hedgehog signaling pathway agonist prior to Day 3 (in the present specification, this method is sometimes referred to as "the production method of the present invention"). The following describes the production method of the present invention.

### Step (1)

Pluripotent stem cells are stem cells capable of being differentiated into all three cell lineages (endoderm, mesoderm, and ectoderm) (i.e., pluripotency) and also having proliferative capacity. Examples of pluripotent stem cells include, but are not limited to, embryonic stem (ES) cells, embryonic stem (ntES) cells derived from cloned embryos obtained by nuclear transfer, sperm stem cells ("GS cells"), embryonic germ cells ("EG cells"), induced pluripotent stem (iPS) cells, and pluripotent cells derived from cultured fibroblasts and bone marrow stem cells (Muse cells). Preferred pluripotent stem cells are ES cells in terms of induction efficiency into suprachiasmatic nucleus organoids and properties and other factors of the resulting suprachiasmatic nucleus organoids. In one embodiment of the present invention, preferred pluripotent stem cells are iPS cells, and more preferably human iPS cells, in terms of availability without destroying embryos, eggs, and the like in the production process.

Methods for producing iPS cells are known in the relevant technical field, and iPS cells can be produced by introducing an initialization factor into any somatic cell. Here, examples of the initialization factor include genes or gene products, such as Oct3/4, Sox2, Sox1, Sox3, Sox15, Sox17, Klf4, Klf2, c-Myc, N-Myc, L-Myc, Nanog, Lin28, Fbx15, ERas, ECAT15-2, Tcl1, beta-catenin, Lin28b, Sall1, Sall4, Esrrb, Nr5a2, Tbx3, and Glis1. These initialization factors may be used singly or in combination. Examples of combinations of initialization factors include combinations described in WO2007/069666, WO2008/118820, WO2009/007852, WO2009/032194, WO2009/058413, WO2009/057831, WO2009/075119, WO2009/079007, WO2009/091659, WO2009/101084, WO2009/101407, WO2009/102983, WO2009/114949, WO2009/117439, WO2009/126250, WO2009/126251, WO2009/126655, WO2009/157593, WO2010/009015, WO2010/033906, WO2010/033920, WO2010/042800, WO2010/050626, WO2010/056831, WO2010/068955, WO2010/098419, WO2010/102267, WO2010/111409, WO2010/111422, WO2010/115050, WO2010/124290, WO2010/147395, WO2010/147612, Huangfu D. et al. (2008), Nat. Biotechnol., 26: 795-797, Shi Y. et al. (2008), Cell Stem Cell, 2: 525-528, Eminli S. et al. (2008), Stem Cells. 26: 2467-2474, Huangfu D. et al. (2008), Nat. Biotechnol. 26: 1269-1275, Shi Y. et al. (2008), Cell Stem Cell, 3: 568-574, Zhao Y. et al. (2008), Cell Stem Cell, 3: 475-479, Marson A., (2008), Cell Stem Cell, 3: 132-135, Feng B. et al. (2009), Nat. Cell Biol. 11: 197-203, R. L. Judson et al., (2009), Nat. Biotechnol., 27: 459-461, Lyssiotis C. A. et al. (2009), Proc Natl Acad Sci USA. 106: 8912-8917, Kim J. B. et al. (2009), Nature. 461: 649-643, Ichida J. K. et al. (2009), Cell Stem Cell. 5: 491-503, Heng J. C. et al. (2010), Cell Stem Cell. 6: 167-74, Han J. et al. (2010), Nature. 463: 1096-100, Mali P. et al. (2010), Stem Cells. 28: 713-720, and Maekawa M. et al. (2011), Nature. 474: 225-9.

Somatic cells include, but are not limited to, any of fetal somatic cells, neonatal somatic cells, and mature healthy or diseased somatic cells, and any of primary cultured cells, subcultured cells, and cell lines. Specific examples of somatic cells include: (1) tissue stem cells (somatic stem cells) such as neural stem cells, hematopoietic stem cells, mesenchymal stem cells, or dental pulp stem cells; (2) tissue progenitor cells; and (3) differentiated cells such as blood cells (such as peripheral blood cells and cord blood cells), lymphocytes, epithelial cells, endothelial cells, muscle cells, fibroblasts (such as skin cells), hair cells, hepatocytes, gastric mucosal cells, intestinal cells, splenocytes, pancreatic cells (such as exocrine pancreatic cells), brain cells, lung cells, kidney cells, and fat cells.

The intermediate medium is a medium having compositional characteristics of an undifferentiated maintenance medium used to maintain undifferentiation of pluripotent stem cells until step (1) and compositional characteristics of a neural differentiation medium used in step (2). It is a medium capable of acclimating pluripotent stem cells to a dedifferentiation environment (culture in a neural differentiation medium). This acclimation contributes to improving the induction efficiency into suprachiasmatic nucleus organoids and the properties and other factors of the resulting suprachiasmatic nucleus organoids.

The undifferentiated maintenance medium is not particularly limited as long as it is a medium capable of maintaining the undifferentiated state of the pluripotent stem cells for a certain period (e.g., 3 days or more, 7 days or more, 14 days or more, or 28 days or more).

The undifferentiated maintenance medium contains an undifferentiated maintenance factor having an action to maintain the undifferentiated state of the pluripotent stem cells. Examples of undifferentiated maintenance factors include FGF-MAPK signal inhibitors (such as PD0325901), Wnt signal activators (GSK3 inhibitors, such as CHIR99021, Bromoindirubin-3'-oxime(BIO), and Kenpaullone), LIF, BMP-4, 6-bromoindirubin-3'-oxime, CDK8/19i, Emricasan, polyamines, Trans-ISRIB Chroman 1, Epiblastin A, GF 109203X, Go6983, IQ-1, PD173074, SU5402, PD184352, PD98059, SB202190, SB216763, sodium butyrate, SP600125, Surfen, U0126, and WH-4-023. These undifferentiated maintenance factors can be used singly or in a combination of two or more.

The concentration of the undifferentiated maintenance factor in the undifferentiated maintenance medium is not limited as long as it is the concentration at which the undifferentiated state can be maintained. For example, the concentration of the FGF-MAPK signal inhibitor is preferably 0.2 to 4 µM, and more preferably 0.5 to 2 µM, and the concentration of the Wnt signal activator is preferably 0.5 to 10 µM, and more preferably 1.5 to 5 µM.

In the present specification, a medium can be prepared using a medium used for culturing animal cells as a basal medium. Examples of the basal medium include Glasgow's Minimal Essential Medium (GMEM), IMDM medium, Medium 199, Eagle's Minimum Essential Medium (EMEM), αMEM medium, Dulbecco's modified Eagle's Medium (DMEM), Ham's F12 medium, RPMI 1640 medium, Fischer's medium, Neurobasal Medium (Life Technologies), and a mixed medium thereof. The medium may contain serum or may be serum-free. If necessary, a medium may contain, for example, one or more serum replacements such as albumin, transferrin, Knockout Serum Replacement (KSR) (serum replacement for FBS during ES cell culture), N2 supplement (Invitrogen), B27 supplement (Invitrogen), a fatty acid, insulin, a collagen precursor, a trace element, 2-mercaptoethanol, and 3'-thiolglycerol, and may also contain one or more substances such as a lipid, an amino acid, L-glutamine, Glutamax (Invitrogen), a non-essential amino acid, a vitamin, a growth factor, a small molecule compound, an antibiotic, an antioxidant, pyruvate, a buffer, and an inorganic salt.

The undifferentiated maintenance medium can also be a commercially available medium prepared by adding necessary components. Examples of such media include StemFit (registered trademark) series AK02N and AK03N.

The neural differentiation medium used in step (2) is not particularly limited as long as it is a medium capable of releasing the undifferentiated state of the cells and inducing neural differentiation.

The neural differentiation medium is free of any undifferentiated maintenance factor that is contained in the undifferentiated maintenance medium, and thus can release the undifferentiated state of the cells and induce neural differentiation.

A basal medium for the neural differentiation medium is preferably IMDM medium, F12 medium, or the like, and more preferably a mixed medium thereof.

Preferably, the neural differentiation medium is a serum-free medium. In this case, the neural differentiation medium may contain a reducing agent, a serum replacement component, and the like. The reducing agent may be preferably thioglycerol, 2-mercaptoethanol, or the like. The serum replacement component may be preferably BSA or the like. More preferably, the neural differentiation medium contains a reducing agent and a serum replacement component in combination. The concentration of the reducing agent (in particular, thioglycerol) is preferably 100 to 1000 µM, and more preferably 300 to 600 µM. The concentration of BSA is preferably 1 to 20 mg/ml, and more preferably 2 to 10 mg/ml.

Preferably, the neural differentiation medium is insulin-free. According to the method of the present invention, even when the neural differentiation medium is an insulin-free medium, it can be used for human cells.

The intermediate medium is typically a medium in which the concentration of an undifferentiated maintenance factor is lower than that in the undifferentiated maintenance medium. It is better when the concentration of an undifferentiated maintenance factor in the intermediate medium is lower. The concentration may be, for example, 70/100 or less, 60/100 or less, 50/100 or less, 40/100 or less, 30/100 or less, 20/100 or less, 10/100 or less, 5/100 or less, 2/100 or less, or 1/100 or less of the concentration of the corresponding undifferentiated maintenance factor in an undifferentiated maintenance medium, for example. The intermediate medium may be free of an undifferentiated maintenance factor.

Preferably, the intermediate medium is a medium whose composition is similar to that of an undifferentiated maintenance factor, except that the concentration is lowered (the change in component concentration is within ±20%, preferably within ±10%, more preferably within ±5%, and even more preferably within ±1%), or a mixed medium of an undifferentiated maintenance medium and a neural differentiation medium.

In one embodiment of the present invention, the concentration of an undifferentiated maintenance factor in the intermediate medium can be lowered stepwise.

Prior to step (3) (described later), the cells are preferably cultured in adhesion in which the cells are cultured while being adhered to a substrate of a culture vessel. For example, the cells can be cultured in adhesion by coating a substrate of a culture vessel with cell adhesion molecules such as an extracellular matrix molecule (specific examples include Matrigel (BD), type I collagen, type IV collagen, gelatin, laminin, heparan sulfate proteoglycan, entactin, and combinations of these) before the substrate is used for culturing.

Regarding culture conditions, the culture temperature is not particularly limited. It is, for example, about 30 to 40°C, and preferably about 36 to 38°C. The cells are cultured in a CO₂-containing air atmosphere. The CO₂ concentration is preferably about 2 to 5%.

The culture period is, for example, 1 to 7 days, preferably 1 to 5 days, more preferably 2 to 5 days, even more preferably 2 to 4 days, and yet even more preferably 3 to 4 days.

### Step (2)

The neural differentiation medium is as described in step (2) above.

Culturing in the differentiation medium allows cell masses that are formed in step (3) (described later) to differentiate into neurons and express suprachiasmatic nucleus markers such as Six3.

Regarding culture conditions, the culture temperature is not particularly limited. It is, for example, 30 to 40°C, preferably about 36 to 38°C. The cell are cultured in a CO₂-containing air atmosphere. The CO₂ concentration is preferably about 2 to 5%.

The culture period is, for example, 4 to 20 days, preferably 4 to 15 days, more preferably 5 to 15 days, even more preferably 5 to 13 days, and yet even more preferably 6 to 13 days.

### (B) Sonic hedgehog signaling pathway agonist

In the production method of the present invention, when the day on which step (2) is started is taken as Day 0, the medium contains a sonic hedgehog signaling pathway agonist prior to Day 3. In other words, a medium that is used during a period from an arbitrary day (a day to start adding a sonic hedgehog signaling pathway agonist) prior to Day 3 to an arbitrary day (a day to finish adding the sonic hedgehog signaling pathway agonist) after Day 3 (i.e., the neural differentiation medium, or each of the neural differentiation medium and the intermediate medium) contains a sonic hedgehog signaling pathway agonist. This makes it possible to promote neural differentiation and improve the induction efficiency into suprachiasmatic nucleus organoids as well as the properties and other factors of the resulting suprachiasmatic nucleus organoids.

The Sonic hedgehog (sometimes described as "Shh" below) signaling pathway agonist is a substance capable of enhancing signal transduction mediated by Shh. Examples of Shh signaling pathway agonists include proteins belonging to the Hedgehog family (e.g., Shh, Ihh), Shh receptor, Shh receptor agonist, Purmorphamine, and smoothened agonist (SAG; 3-chloro-N-[trans-4-(methylamino)cyclohexyl]-N-[[3-(4-pyridinyl)phenyl]methyl]-benzo[b]thiophene-2-carboxamide). A preferred Shh signaling pathway agonist is SAG.

The sonic hedgehog signaling promoting activity of SAG can be determined by a method known to a person skilled in the relevant technical field, such as reporter gene assay focusing on Gli1 gene expression (Oncogene (2007) 26, 5163-5168).

The concentration of SAG in the medium is, for example, 0.001 µM or more, preferably 0.01 µM or more, more preferably 0.1 µM or more, even more preferably 0.2 µM or more, and yet even more preferably 0.5 µM or more. The concentration is preferably 0.2 to 5 µM, more preferably 0.5 to 2 µM, and even more preferably 0.7 to 1.5 µM. When a sonic hedgehog signaling pathway agonist other than SAG is used, preferably, it is used at a concentration at which it shows sonic hedgehog signaling promoting activity equivalent to that of SAG at the above concentrations.

The day to start adding a sonic hedgehog signaling pathway agonist is preferably within the period from Day -6 until Day 3, more preferably within the period from Day -4 until Day 3, even more preferably within the period from Day -2 until Day 3, yet even more preferably within the period from Day 0 until Day 3, especially preferably within the period from Day 1 until Day 3, further especially preferably within the period from Day 2 until Day 3, and particularly preferably Day 2.

The day to finish the sonic hedgehog signaling pathway agonist is, for example, within the period from Day 4 until Day 100, preferably within the period from Day 4 until Day 20, more preferably within the period from Day 4 until Day 15, even more preferably within the period from Day 5 until Day 13, yet even more preferably within the period from Day 6 until Day 13, and particularly preferably the day on which step (2) is finished.

### Step (3)

The timing to start the operation for cell mass formation is not limited as long as it is prior to the start of step (2). In one embodiment of the present invention, the timing is not particularly limited as long as it is within the period from the start of step (1) until the start of step (2); however, it is preferably within the period from Day -6 before Day 0, more preferably within the period from Day -5 before Day 0, even more preferably within the period from Day -4 before Day 0, and yet even more preferably within the period from Day -3 before Day 0.

In the case of adhesion culture, the cells are removed from a culture substrate and dispersed in the medium by pipetting or the like according to or based on a known method before the operation for cell mass formation.

The operation for cell mass formation is not particularly limited as long as it allows cells to aggregate and form a cell mass having a diameter of 100 µm or more (preferably 200 µm or more, particularly preferably 500 µm or more). For example, the operation includes seeding cells into culture wells and culturing the cells in suspension, or centrifuging a cell suspension. The former is particularly preferred.

When seeding cells into a culture substrate and culturing the cells in suspension, the dispersed cells may be seeded into a relatively large culture vessel, such as a 10 cm dish, to simultaneously form multiple cell masses in one culture vessel; however, in this case, the size of each cell mass varies. Thus, for example, a certain number of dispersed cells are placed in culture wells (each well of a multiwell plate with U-bottom or V-bottom, such as a 96-well microplate) and cultured statically, so the cells aggregate quickly and form one aggregate in each well. Such aggregates are collected from multiple wells, whereby a uniform population of aggregates can be obtained.

A non-cell-adhesive culture vessel is preferred to enable suspension culture. Usable non-cell-adhesive culture vessels include culture vessels whose surfaces are not artificially treated for improving adhesiveness to the cells (e.g., coating treatment with extracellular matrix such as basement membrane preparation, laminin, entactin, collagen, and gelatin, or with polymer such as polylysine and polyornithine; or surface treatment such as positive electric charge treatment). As a non-cell-adhesive culture vessel, for example, a culture vessels whose surface has been artificially treated to decrease adhesiveness to the cells (e.g., superhydrophilic treatment with MPC polymer etc., and protein low adsorption treatment) can be used.

The duration from the operation for cell mass formation to the completion of cell mass formation is preferably within three days, more preferably within two days, even more preferably within one day, and particularly preferably 12 hours to 24 hours. Conditions for the operation for cell mass formation (e.g., the area, shape, etc. of the culture bottom) can be adjusted to adjust the duration.

In step (1) and step (2), the cell mass formation is directly followed by suspension culture.

### (A) Number of raw material cells in cell mass

In the production method of the present invention, the number of raw material cells seeded to form one cell mass in step (3) is 6000 or greater. This makes it possible to promote neural differentiation and improve the induction efficiency into suprachiasmatic nucleus organoids as well as the properties and other factors of the resulting suprachiasmatic nucleus organoids.

The number of raw material cells is preferably 9000 or greater, more preferably 12000 or greater, even more preferably 15000 or greater, and yet even more preferably 20000 or greater. The upper limit is not particularly limited. For example, the number is 200000, 100000, or 80000.

### Step (4)

Preferably, the production method of the present invention further includes (4) a step of culturing cell masses obtained in step (2) in a neural differentiation medium. This makes it possible to further promote maturation of suprachiasmatic nucleus organoids.

Preferably, step (4) is performed under high oxygen conditions. Specifically, in step (4), the cells are preferably cultured in an incubator in a high-oxygen concentration atmosphere. The high-oxygen concentration may be any concentration higher than the oxygen concentration in the air, but it is preferably 25 to 60%, more preferably 30 to 50%, and particularly preferably 35 to 45%.

Alternatively, step (4) can be performed under semi-aerobic culture conditions. Specifically, in one example method, the cell mass is placed on a Millicell or Transwell, with the lower surface being immersed in an expansion medium and/or maturation medium and the upper surface being in contact with air.

Preferably, step (4) includes a step of culturing using an expansion medium as a neural differentiation medium and a step of culturing using a maturation medium as a neural differentiation medium.

A basal medium for the expansion medium is preferably DMEM medium, F12 medium, or the like, and more preferably a mixed medium thereof.

Preferably, the expansion medium is a serum-free medium. In this case, the neural differentiation medium contains a serum replacement component. The serum replacement component may be preferably KSR or the like. The concentration of KSR is preferably 5 to 20%, and more preferably 7 to 15%.

The culture period in the expansion medium is, for example, 4 to 20 days, preferably 4 to 15 days, more preferably 5 to 15 days, even more preferably 5 to 13 days, and yet even more preferably 6 to 13 days.

A basal medium for the maturation medium is preferably DMEM medium, F12 medium, or the like, and more preferably a mixed medium thereof.

The maturation medium is preferably a serum-free medium. In this case, the neural differentiation medium contains a serum replacement component. The serum replacement component may be preferably N2 supplement, B27 supplement, or the like, and more preferably a combination of these. The concentration of N2 supplement is preferably 0.2 to 5%, and more preferably 0.5 to 2%. The concentration of B27 supplement is preferably 0.5 to 10%, and more preferably 1 to 3%.

The culture period in the maturation medium is, for example, 4 to 400 days.

In step (4), the cells can be cultured in a medium containing a sonic hedgehog signaling pathway agonist or a medium free of a sonic hedgehog signaling pathway agonist.

The production method of the present invention can produce suprachiasmatic nucleus organoids having a high proportion of suprachiasmatic nucleus cells. The proportion is, for example, 1% or more, preferably 5% or more, more preferably 10% or more, even more preferably 15% or more, and yet even more preferably 18% or more. The upper limit is not particularly limited, and may be, for example, 50%, 40%, or 30%. The proportion can be measured according to Test Example 1-3 (described later).

The production method of the present invention can produce suprachiasmatic nucleus organoids in which all constituent cells are brain tissue cells. Examples of brain tissue cells include excitatory neurons, inhibitory neurons, meninges, and glial cells. The constituent cells can be determined according to Test Example 1-3 (described later).

### 2. Screening method

In one embodiment, the present invention relates to a method for screening for a circadian rhythm regulator, including: (X) a step of contacting a suprachiasmatic nucleus organoid in which the proportion of suprachiasmatic nucleus cells is 5% or more (preferably 10% or more, more preferably 15% or more, and even more preferably 18% or more) or the suprachiasmatic nucleus organoid obtained by the production method of the present invention with a test substance; and (Y) a step of evaluating the circadian rhythm in the suprachiasmatic nucleus organoid. The following describes the screening method.

Examples of test substances include cell extracts, cell culture supernatants, microbial fermentation products, marine organism-derived extracts, plant extracts, purified or crude proteins, peptides, non-peptide compounds, synthetic small molecule compounds, and natural compounds.

Such a test substance can also be obtained using any of many approaches in combinatorial library methods known in the relevant technical field, including (1) a biological library, (2) a synthetic library method using deconvolution, (3) a "one-bead one-compound" library method, and (4) a synthetic library method using affinity chromatography separation. Application of a biological library method using affinity chromatography separation is limited to peptide libraries, but the other four approaches are applicable to small molecule compound libraries of peptides, non-peptide oligomers, or compounds (Lam (1997) Anticancer Drug Des. 12: 145-67). Examples of methods for synthesizing molecular libraries can be found in the relevant technical field (DeWitt et al. (1993) Proc. Natl. Acad. Sci. USA 90: 6909-13; Erb et al. (1994) Proc. Natl. Acad. Sci. USA 91: 11422-6; Zuckermann et al. (1994) J. Med. Chem. 37: 2678-85; Cho et al. (1993) Science 261: 1303-5; Carell et al. (1994) Angew. Chem. Int. Ed. Engl. 33: 2059; Carell et al. (1994) Angew. Chem. Int. Ed. Engl. 33: 2061; and Gallop et al. (1994) J. Med. Chem. 37: 1233-51). Compound libraries can be made as a solution (see Houghten (1992) Bio/Techniques 13: 412-21) or beads (Lam (1991) Nature 354: 82-4), chips (Fodor (1993) Nature 364: 555-6), bacteria (U.S. Pat. No. 5,223,409), spores (U.S. Pat. Nos. 5,571,698, 5,403,484, and 5,223,409), plasmid (Cull et al. (1992) Proc. Natl. Acad. Sci. USA 89: 1865-9), or phage (Scott and Smith (1990) Science 249: 386-90; Devlin (1990) Science 249: 404-6; Cwirla et al. (1990) Proc. Natl. Acad. Sci. USA 87: 6378-82; Felici (1991) J. Mol. Biol. 222: 301-10; and U.S. Patent Application No. 2002103360).

Step (X) can be performed, for example, by adding a test substance to a medium containing suprachiasmatic nucleus organoids. The concentration of the test substance in the medium can be appropriately set according to the type of the test substance and other factors.

The period of contact with the test substance is not particularly limited. It can be, for example, 1 hour to 14 days.

Step (Y) can be performed, for example, by measuring clock gene expression levels in the suprachiasmatic nucleus organoids and evaluating the fluctuation or oscillation of expression levels or persistence of the fluctuation or oscillation. The measurement and evaluation can be performed according to or based on known methods.

The circadian rhythm in the suprachiasmatic nucleus organoids in step (Y) can be evaluated by, for example, measuring the fluctuation or oscillation of expression levels of receptors expressed in the suprachiasmatic nucleus organoids or persistence of the fluctuations or oscillation as well as responsiveness of these receptors (e.g., the responsiveness in the presence or absence of the test substance, or the responsiveness to ligands in the presence or absence of the test substance). The receptors are not particularly limited as long as they are expressed in the suprachiasmatic nucleus organoids. Examples thereof are given below in descending order of expression levels: Bsg, Rora, Prnp, Cd47, Gria2, Rack1, Nrxn1, Epha5, Thra, Ogfrl1, Rorb, Nrxn2, Gabbr1, Sqstm1, Atp6ap2, Ntrk2, Slc22a17, Grin2b, Nrp2, Vldlr, Gabrb3, Edil3, Unc5c, Nr1d1, Adgrl1, Crlf2, Nlgn1, Tmem63b, Nlgn2, Grm5, Stat3, Nr2f2, Jmjd6, Bmpr2, Tm2d1, Gabrg3, Fzd3, Ntrk3, Gpr162, Nptxr, L1cam, Lrp1, Kit, Adgrb3, Amfr, Nr4a1, Sorl1, Tex264, Csf2ra, Grin1, Glrb, Ptprf, Sema6a, Vipr2, Gabra2, Acvr2a, Grik5, Nrld2, Adgrb1, Gabrb1, Gpr27, Rgmb, Lrp8, Gria4, Adipor1, Mfsd6, Unc5d, Ptch1, Cntfr, Ogfr, Palld, Plxnc1, Adgrl3, Adgra1, Sdc4, Nr2c2, Cry2, Paqr8, Gabrg1, Ephb1, Atrn, Gabre, Igflr, Ddr1, Gabra1, Gria1, Lgals3, Nr4a3, Grpr, Celsr2, Neo1, Cry1, Gabrg2, Ednrb, Lrp6, Extl3, Drd1, Gprc5b, Adipor2, Igf2r, Gipr, Epha4, Nrp1, Il6st, Plxnb2, Gabra3, Ryk, Insr, Gpr85, Sema5a, Nr2f6, Ephb2, Pkd1, Bmprla, Nr3c1, Gpr19, Gpr158, Rtn4rl1, Npbwr1, Adgrg1, Acvr1b, Grm1, Avprla, Lmbr1, Smo, Plxna1, Gpr153, Sort1, Adcyap1r1, Mfge8, Grik2, Plxna2, Gabbr2, Gfra4, Acvr2b, Gfra2, Paqr9, Nlgn3, Ar, Grik1, Adgrb2, Oprl1, Rxrb, Paqr7, Unc13b, B9d1, Grin3a, Crcp, Gabrq, Scarb1, Grik3, Amot, Styk1, Fgfr1, Arnt, Sigmar1, Chrnb2, Unc5a, Gabrb2, Agtrap, Adgr12, Nrlh2, Ifnar1, Ifngr2, Npy1r, Slc39a9, Tnfrsfla, Plxna4, Sema4d, Fzd8, Ifngr1, Il11ra1, Nrg2, P2rx4, Nr2f1, Epha8, Ahr, Cnr1, Grin2a, Npy6r, Esrra, Abca1, Erbb4, Fzd5, Gpr22, Lpar6, Plxnb1, Prokr2, Ptprm, Inpp5k, Gpr45, Tpra1, Nectin1, Tgfbr1, Ptprt, Paqr4, Mrgpre, Cr1l, Gfra1, Ackr1, Gpr176, P2ry1, Ghsr, Gpr135, Celsr3, Pgr, Il1rap, Rgma, Rxra, Nr4a2, Slc52a2, Ephb6, Rarb, Gabra5, Gpr61, Dcbld1, Plxna3, Grid1, Hrh3, Tyro3, Grik4, Srebf1, Lgr4, Epha6, Adralb, Npr2, Dhx16, Gria3, Ghr, Ramp2, Abca7, Adrb1, Ntsr2, Tspan12, Dcc, S1pr3, Ppard, Oprk1, Pdgfra, Grid2, Dcbld2, Abhd2, Sphk2, Lifr, Rara, Gpr179, F3, Gpr101, Gpr37l1, Cp, Adgrv1, Robo1, Lpar1, Gal, Paqr3, Nradd, Grm8, Notch2, Epha7, Calcr, Ramp1, Grin2d, Nid1, Acvr1, Alk, Gpr75, Glra3, Notch1, F2r, Grm3, Fzd6, Il13ra1, Nr6a1, Robo2, Npy5r, Gpr173, Gpr165, Cd44, Sstr1, Fzd1, Gpr6, Nr2c1, Gabra4, Grm7, Ifnar2, Gpr161, Gpr17, Pglyrp1, F8, Adrala, S1pr1, Ret, P2ry14, Cd200r2, Il3ra, Tmem63c, Adora1, Gpr146, Pgr15l, Lrp4, Glra2, Hrh1, Thrb, Fzd2, Prlr, Hpgd, Ebi3, Tspo, Lbp, Lmbr1l, Kiss1r, Egfr, Gpr12, Igsf1, Unc13c, Rtbdn, Ltk, Gpr37, Antxr1, Hspg2, Ctsh, Htr2c, Adgrg6, Gprc5c, Colec12, Plxnd1, Epha10, Tgfbr3, Reg3g, Rorc, Htr5a, Tnfrsf19, Fzd4, Pparg, Reck, Mdga1, Cd302, Fgfr3, Nr3c2, Folr1, S1pr2, Ptk7, P2rx7, Fgfr2, Grm4, Agtr1a, Chrm2, Celsr1, Ntsr1, Lgr5, Stra6, Rtn4r, Chrna3, Chrm3, Esrrg, Chrna4, Adra2a, Bmprlb, Fas, Mrc2, Gpr26, Met, Gpr83, Pde3a, S1pr5, Unc5b, Trhr, P2rx6, Ager, Esr1, Drd2, Adgra3, Cd27, Prtg, Epha3, Gpr150, Ly96, Crhr1, Glplr, Htr1b, Gpr21, Flt1, Sstr2, Cspg4, Il10rb, Il6ra, Adgre5, Ednra, Adora2a, Nod1, Npr1, Pthlr, Tmem116, Ephb3, Grin2c, Gpr68, Tgfbr2, Acvrlc, Gpr62, Fgfrl1, Gpr139, Npy2r, Lag3, Egf, Lpar2, Asgr1, Opn3, Kdr, Fzd9, Adgrg2, Nmur2, Cd36, Cr2, Lrp5, Htr2a, Rxrg, Cxcr4, Axl, Il4ra, Tshr, Ddr2, Tnfrsf10b, Il15ra, Ptger3, Cd200r4, Notch3, Hcrtr2, Eda2r, Sostdc1, Epor, Gpr149, Npffr1, Efemp1, Vdr, Itga2, Htr7, Il1r2, Rarg, Gpr50, Il17rd, Aim2, Crlf1, Flt3, Mchr1, Vmn2r1, Ramp3, Gpr88, Il1rapl2, Tnfrsflb, Tacr1, Tnfrsf23, Chrnb1, Cd200r1, Cx3cr1, Gpr3, Adora2b, Trim12a, Notch4, Fzd7, Csflr, Qrfprl, Gucy2e, Scn7a, Il17ra, Rxfp1, Nr2e1, Il17rc, Gpr157, Trim12c, Adra2c, Gper1, Oxtr, Ptch2, Hcrtr1, Adgrf4, Paqr6, Calcrl, Nr5a2, Ccr10, Pdgfrb, Sstr3, Plxnb3, Ackr3, Insrr, Htr1a, Lpar4, Osmr, Ppara, Grm2, Chrm4, Oprm1, Chrna5, Tgfbr3l, Tmem63a, Cd74, Adrb2, Trim30d, Lgr6, F2rl3, Tacr3, Chrng, Cd14, Qrfpr, Fcgr1, Chrna7, Hmcn2, Gpr55, Gpr35, Nfam1, Tnfrsf22, Adgra2, Cckbr, Vmn2r3, Tnfrsf4, Thbd, Adgrf5, Ntrk1, Ngfr, Cckar, Erbb2, Adgrg3, Sphk1, Fzd10, Chrm5, Fcer2a, Chrnb4, Opn1mw, F2rl2, Vmn2r56, Chrnb3, Mertk, Procr, Ror2, Htr6, Nrlh3, Anxa9, Gpr84, Htr3a, Tlr3, Cd79a, Gucy2c, Ephb4, Mc1r, Tnfrsf18, Gpr25, Hmcn1, Glra1, Adrb3, Slamf1, Cd40, Amhr2, Trpv1, Eng, Antxr2, Epha2, Chrna9, Il1r1, Tlr2, Trbc2, Npr3, Lpar3, Hrh2, Htrld, Ifih1, Ccrl2, Gabrd, Adgrg5, Lepr, Gabrr2, Gpr34, Gpr156, Tnfrsf9, Vmn2r57, Il17re, C3ar1, Il20ra, Pirb, Htr2b, Pecam1, Gpr82, Gpr87, Mstlr, Tek, Gpr160, Brs3, Grin3b, Nlrp6, Cd72, Edar, Ror1, Adora3, Galr1, Tlr5, Ffar4, Ly75, Cd300lg, Crhr2, Fcrlb, C5ar2, Chrna6, Acvrl1, Gpr52, Klri2, Tnfrsf13c, Cd247, P2ry2, Gprc5a, Fcamr, Oscar, Prlhr, Nod2, Chrm1, Taslr3, Dmbt1, Pigr, Htrlf, Klrk1, Il17rb, Bdkrb1, Pearl, Chrna1, Trim30a, Adgre1, Cd80, Fcerlg, Nrli3, Btla, Il31ra, Ffar2, Tnfrsf11a, Galr2, Ptger4, Oprd1, Kir3dl2, Il22ra1, Htr4, Prokr1, Rgr, F2, Ptgfr, F2rl1, Klrblf, Rxfp3, Il2ra, Bdkrb2, Ackr4, Il10ra, Itga11, Rxfp2, Cubn, Ptger1, Il12rb2, Mc4r, Il20rb, Asgr2, Vmn2r120, Vmnlr43, Ptger2, Rho, Npffr2, Gpr4, Fcgr2b, Lyve1, Cysltr2, Drd5, Ccr5, Vmn2r79, P2ry12, Ccr2, Cmklr1, Mc3r, Vmn2r113, Paqr5, P2rx2, Ahrr, Npy4r, Gucy2f, Adgrf3, Hcar2, Folr2, Mc5r, Fgfr4, Rrh, Csf2rb, Gabrr1, Gpr152, Ptgdr, Fcgr4, Clecla, Htr3b, Erbb3, Trpa1, Tnfrsf8, Epha1, Fcmr, Ltb4r1, Abcc9, Agtr2, Gpbar1, Hcar1, Ptpn6, Ackr2, Gpr63, Nmbr, P2ry6, Sstr5, Mrgprf, Ltb4r2, Drd3, Htr5b, Treml1, Gpr171, Ifnlr1, Trim5, Casr, Klrbla, Gpr18, Gabrp, Mrc1, Drd4, Gcgr, Il2rb, Fcna, Ptgir, Opnlsw, Itgb2, Il18r1, Vmn2r116, Nrli2, Pla2r1, Chrne, Hephl1, Cxcr2, Vipr1, Sctr, Musk, Cd300lb, Sucnr1, Il12rb1, Cysltr1, Vmn2r84, P2rx3, Lrrc19, Vmn2r86, Vmn2r27, Tlr4, Nr5a1, Cel, Uts2r, Gpr20, Chrna2, Gpr39, Tas2r137, Trpv4, Klrblc, Vmn2r29, Mtnrla, Esrrb, Clec10a, Adgrd1, Hnf4a, Gfral, Unc5cl, Il2rg, Pth2r, Galr3, Cd300a, Tbxa2r, Gpr182, Ighm, Ccr9, Hrh4, Il21r, Mc2r, Ly6g6e, Hjv, Gnrhr, Aplnr, Tlr6, Il1rl2, Hnf4g, Itgal, Il1rl1, Sstr4, Opn4, Ffar1, Gpr65, Glp2r, Vmn2r59, Flt4, Tas2r135, Fpr2, Trem2, Tas1r1, Gabra6, Vmn1r41, Tlr12, Gpr183, Vmn2r53, Vmn2r78, Vmn2r85, Fcgr3, Vmn2r87, Adgrf1, Vmn2r6, Il5ra, Trbc1, Fpr1, Vmn2r124, Itgb2l, F5, Gpr141, Mpl, Glra4, Fcrl1, Grm6, Rtn4rl2, Nrlh5, Tas2r108, Spn, Cd300e, Csf2rb2, Klre1, Adra2b, Klrb1, Klrd1, Slamf8, Cd4, Clec12a, Gprc5d, Esr2, Pkd2l1, C5ar1, I127ra, Cd79b, Chrna10, Gm7609, Reg3b, Gpr151, Trac, Clec4d, Csf3r, Ptafr, Tlr13, Ffar3, Cxcr6, Il12b, Vmn1r42, Cxcr5, Vmn2r115, Taslr2, Nlrplb, Adgrf2, Ccr1, Vmnlr54, Vmn2r26, Trhr2, Cd3g, Tas2r143, Pglyrp4, Vmn2r5, Cnr2, Npsr1, Vmn1r40, Tie1, Adgrl4, Vmnlr53, Chrnd, Il13ra2, Vmn2r24, Gucy2g, Vmn2r23, Opn5, P2rx1, Il18rap, Il11ra2, Tmigd3, Tnfrsf17, Ptgdr2, Ms4a2, Lpar5, Vmn2r69, Vmn2r25, P2ry10b, Tacr2, Nr2e3, Ccr6, Cleclb, Gpr174, Agtrlb, Vmn2r108, Pkd1l3, Il9r, Xcr1, Il7r, Vmn2r22, Taar4, Vmnlr49, Stra6l, Vmn2r54, Adrald, Adgre4, Vmn2r52, Gfra3, Treml4, P2ry4, Robo4, Ghrhr, Vmn2r111, Gpr119, Nrlh4, Cd200r3, Vmn2r93, Tlr7, Ccr7, Vmn2r95, Trim30b, Nmur1, Vmn2r89, Reg1, Vmn2r18, Vmn2r97, Ccr4, Vmn2r118, Vmn2r83, Cxcr1, Cd300lf, P2ry13, Cd7, Reg2, Vmn1r47, Gpr15, Cd8b1, Gprc6a, Lhcgr, Vmn2r17, and Mrgprh. Other examples include Il23r, Klrblb, Vmn2r96, Avpr2, Cd160, Gpr155, and Nkx3-1. These receptors have been confirmed to be expressed in the suprachiasmatic nucleus organoids of the present invention.

The circadian rhythm in the suprachiasmatic nucleus organoids in step (Y) can be evaluated by, for example, combining the following: measuring clock gene expression levels and evaluating the fluctuation or oscillation of the expression levels or persistence of the fluctuation or oscillation; and measuring the fluctuation or oscillation of expression levels of receptors expressed in the suprachiasmatic nucleus organoids or persistence of the fluctuations or oscillation as well as responsiveness of these receptors (e.g., the responsiveness in the presence or absence of the test substance, or the responsiveness to ligands in the presence or absence of the test substance). For example, the evaluation can be performed by measuring the receptor responsive upon ligand addition during fluctuations in clock gene expression levels.

As a result of the evaluation in step (Y), when a change in the circadian rhythm was observed compared to the control samples evaluated under the same conditions except that the test substance was not used, the test substance can be selected as a circadian rhythm regulator (or its candidate substance).

The selected circadian rhythm regulator can be used as an active ingredient in an agent for treating, ameliorating, or preventing diseases, disorders, etc., in which the circadian rhythm is involved. Examples of such drugs include sleeping pills.

### Examples

The present invention is specifically described with reference to the Examples, which do not limit the present invention.

### Test Example 1. Suprachiasmatic nucleus organoid production 1

### Test Example 1-1. Culturing

Culture conditions were examined and determined using persistent Six3 positivity as an indicator.

Mouse ES cells (C57/B6N Per2::Luciferase cells (Scientific Reports, vol. 6, Article number: 32769, 2016; Nature Protocols, vol. 12, pp 2513-2530, 2017) or Six3::mVenus cells (Nature Communications, vol. 8, Article number: 1339, 2017)) were cultured in adhesion in a feeder-free manner in an undifferentiated maintenance medium (composition: Glasgow Minimum Essential Medium (GMEM, Gibco); 10% KnockOut^{™} Serum Replacement (KSR; Gibco); 1% foetal bovine serum (FBS); 1 mM sodium pyruvate (Gibco); 1 × MEM Non-Essential Amino Acids (Gibco); and 100 µM β-mercaptoethanol (Wako, β-ME), with the addition of two inhibitors (2i) (3 µM CHIR99021 (Axon) (Wnt signal activator) and 1 µM PD0325901 (Wako) (FGF-MAPK signal inhibitor)) and the addition of ESGRO mouse leukaemia inhibitory factor (mLIF, Millipore) to a final concentration of 2 × 10³ U/mL). The medium was changed to an intermediate medium (having the same composition as that of the undifferentiated maintenance medium, except for excluding the FGF-MAPK signal inhibitor and the Wnt signal activator). The cells were cultured in the intermediate medium for 3 days.

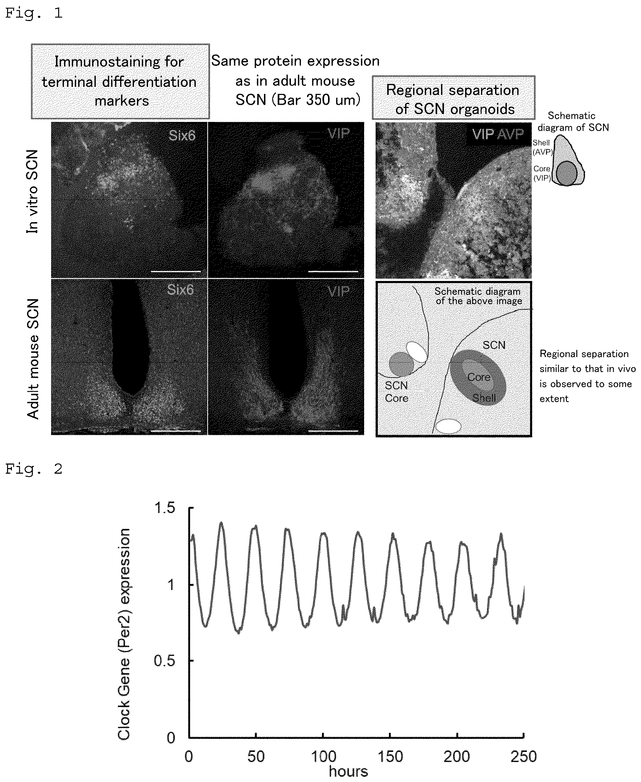

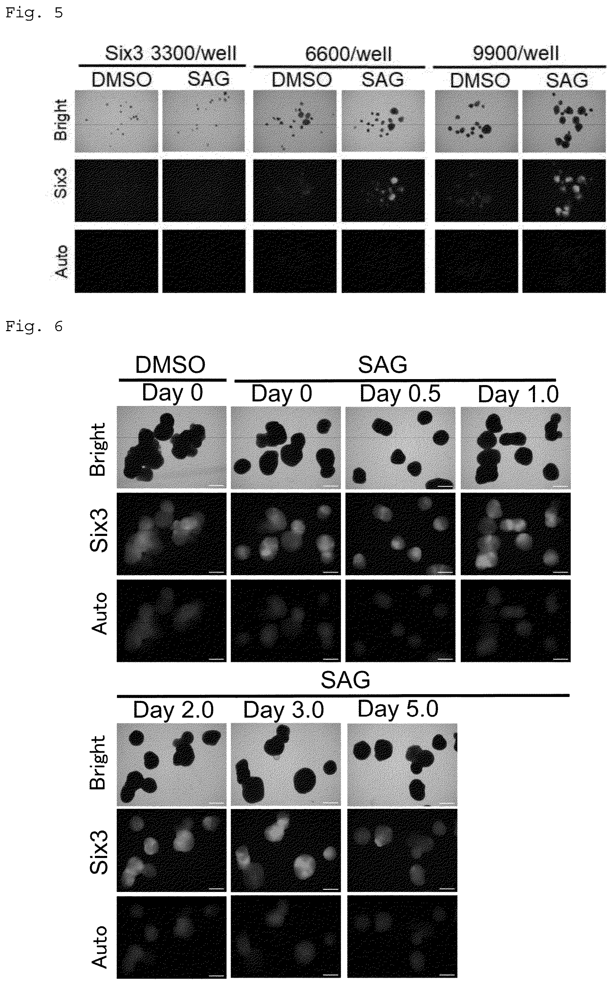

The cells were collected and seeded into a 96-well plate (U-bottom well) at 30000 cells/well (in the case of Per2::Luciferase cells; used in the tests shown in Figs. 1 to 4 and 7) or at 10000 cells/well (in the case of Six3::mVenus cells; used in the tests shown in Figs. 5 and 6), and culturing started in a neural differentiation medium (composition: Iscove's modified Dulbecco's medium/Ham's F-12 1:1, 1× chemically defined lipid concentrate, penicillin/streptomycin, monothioglycerol (450 µM), and purified BSA (>99% purified by crystallization; Sigma, 5 mg/ml)) (Day 0). On Day 1, the formation of one spherical cell mass (diameter: about 500 µM) was observed in each well. On Day 2, a sonic hedgehog agonist (SAG, LX-270-426-M001 available from Enzo Biochem Inc.) was added to a final concentration of 1 µM. Culturing continued until Day 7.

On Day 7, the cell masses were collected, washed once with a neural differentiation medium (expansion medium) (composition: DMEM/F-12, GlutaMAX^{™} supplement, 10% KSR), and then seeded into an EZSphere or the like (culture dish) to start suspension culture in the neural differentiation medium (expansion medium). Culturing was performed in a multi-gas incubator with 40% O₂ and 5% CO₂ and continued until Day 13. The medium was replaced every other day.

On Day 13, the medium was changed to a neural differentiation medium (maturation medium) (composition: DMEM/F-12, GlutaMAX^{™} supplement, N2 supplements 1%, B27 (with Vitamin A) 2%), and the cells were continuously cultured in suspension. Culturing continued in the multi-gas incubator with 40% O₂ and 5% CO₂. The medium was replaced every other day.

Within the period from Day 20 until Day 30, the cell masses were observed under a phase contrast microscope, and cell masses with transparent protrusions on their surfaces (fluffy aggregates) were removed. Although it depends on the batch, about 10 to 50% of the cell masses were fluffy aggregates.

The cell masses were collected, and organoids were obtained.

### Test Example 1-2. Immunostaining

The organoids (Day 72) obtained in Test Example 1-1 were immunostained with terminal differentiation markers (Six6, VIP, and AVP) of the suprachiasmatic nucleus. For comparison, the suprachiasmatic nuclei of adult mice were immunostained in the same manner. Immunostaining was performed according to a known method.

Fig. 1 shows the results. The organoids expressed terminal differentiation markers of the suprachiasmatic nucleus in the same manner as the suprachiasmatic nuclei of adult mice, and formed a regional distribution similar to that of the suprachiasmatic nuclei of adult mice to some extent.

### Test Example 1-3. Single-cell RNA-seq analysis

The organoids (Day 70) obtained in Test Example 1-1 were subjected to single-cell RNA-Seq analysis to calculate the proportion of suprachiasmatic nucleus cells in the organoid constituent cells.

A specific method is as follows. 11 SCN organoid cell masses at Day 70 were dispersed using a neuron dissociation solution, and large masses were removed using Flowmi. After thorough washing, trypan blue was used to confirm cell viability of at least 80%, and a library was prepared using the Chromium v3.1 kit (10x genomics), targeting 5,000 cells. Subsequently, sequencing was performed using Illumina Novaseq, and analysis was performed using Cellranger and Seurat.

The main populations were roughly half neurons and half glia, with the rest consisting of ependyma, pia mater, and oligodendrocytes, representing a complete set of cells from the surface to the interior of the brain. Only seven neuronal populations were identified, of which only four were major populations. Annotation was performed based on scRNA-Seq data from the mouse hypothalamus (Romanov Nature 2020), which found that the four major populations were SCN, Arc-TIDA (dopamine neurons in the arcuate nucleus), Arc-Agrp (e.g., Agrp neurons in the arcuate nucleus), and Lh-Hcrt (e.g., orexin neurons in the lateral field). Only a small portion of the hypothalamus was targeted for induction. The table below shows the specific proportion relative to the in vivo cell ratios, based on the Mano Cell Report Method (https://www.cell.com/cell-reports-methods/pdfExtended/S2667-2375(21)00083-7).

**[Table 1]**

| | SCN Organoids(%) | Adult hypothalamus (%) | Concentration rate |
|---|---|---|---|
| SCN | 20.58 | 0.70 | 29.45 |
| Arc | 44.12 | 1.54 | 28.70 |
| LH | 20.91 | 12.51 | 1.67 |

### Test Example 1-4. Clock gene expression analysis

The organoids obtained in Test Example 1-1 were subjected to clock gene expression analysis to evaluate the persistence of circadian oscillation.

A specific method is as follows. The SCN organoids produced using Per2::Luciferase KI/KI cells were embedded in agarose gel at the bottom of a 35 mm glass-bottom dish, and the medium was replaced with a phenol red-free maturation medium. From Day 101, 500 uM of Luciferin was added and measurements were taken with a 20× objective lens and a 0.5× magnification lens, using an LCV 100 (Olympus) with modified lighting, under the following imaging conditions: 16-bit, EM gain 500, exposure 50 min, binning 3, every hour.

Fig. 2 shows the results. It was found that the oscillation of clock genes lasted for 10 days or more. Since the peak-to-trough ratio remained constant, only a decrease in the baseline due to substrate consumption and other factors was observed, and no attenuation from desynchronization was observed.

### Test Example 1-5. Transplant analysis

The organoids obtained in Test Example 1-1 were transplanted into mice with disrupted suprachiasmatic nuclei, and movements of the mice before and after transplantation were measured to produce an actogram and a chi-squared periodogram.

A specific method is as follows. SCN disruption and transplantation experiments were performed according to previous literature (Sujino Curr Biol 2006; https://www.sciencedirect.com/science/article/pii/S09609822030022 27). SCN organoids were transplanted instead of mouse fetal hypothalamus. The SCN organoids at Day 20 were placed on a Millicell under semi-aerobic conditions and cultured overnight in a CO₂ incubator (5% CO₂ and 20% O₂). Then, the SCN organoids were cut into 3 to 4 sections using ophthalmic scissors and transplanted into SCN-disrupted mice as described in the previous literature, and the wheel-spinning rhythm was analyzed.

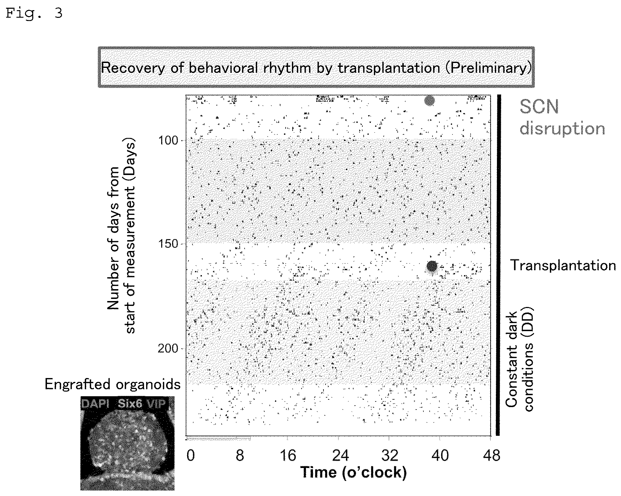

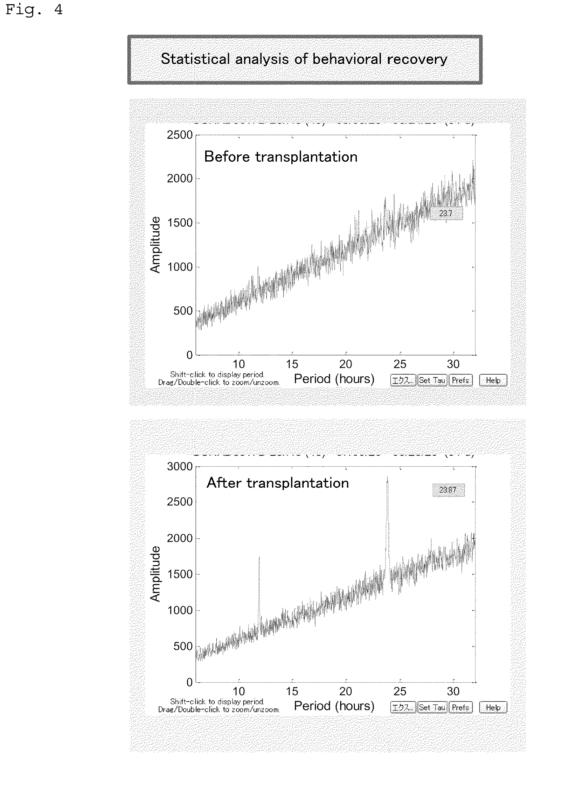

Figs. 3 and 4 show the results. The rhythm that had disappeared due to suprachiasmatic nucleus disruption was recovered after transplantation of the suprachiasmatic nucleus organoids.

### Test Example 2. Suprachiasmatic nucleus organoid production 2

Culturing was performed in the same manner as in Test Example 1, except that the number of cells seeded on Day 0 was 3300 cells/well, 6600 cells/well, or 9900 cells/well, and that a SAG-non-added group (DMSO-added group) was also obtained.

Fig. 5 shows brightfield images (Bright), fluorescence images (showing Six3 expression), and autofluorescence images (Auto) of the cell masses at Day 13. It was found that the suprachiasmatic nucleus markers were well expressed in the organoids when the number of raw material cells seeded to form one cell mass was 6000 or greater.

### Test Example 3. Suprachiasmatic nucleus organoid production 3

Culturing was performed in the same manner as in Test Example 1, except that SAG was added on Day 0, Day 0.5, Day 1.0, Day 2.0, Day 3.0, or Day 5.0, and that a SAG-non-added group (DMSO-added group at Day 0) was also obtained.

Fig. 6 shows brightfield image (Bright), fluorescence images (showing Six3 expression), and autofluorescence images (Auto) of cell masses at Day 48. It was found that the suprachiasmatic nucleus markers were well expressed in the organoids when SAG was added prior to Day 3.

### Test Example 4. Suprachiasmatic nucleus organoid production 4

Culturing was performed in the same manner as in Test Example 1, except that the number of cells seeded on Day 0 was 30000 cells/well, 45000 cells/well, or 60000 cells/well, that a group (2i(+) maintenance) that uses a undifferentiated maintenance medium instead of the intermediate medium was also obtained, and that a SAG-non-added group (DMSO-added group) was also obtained.

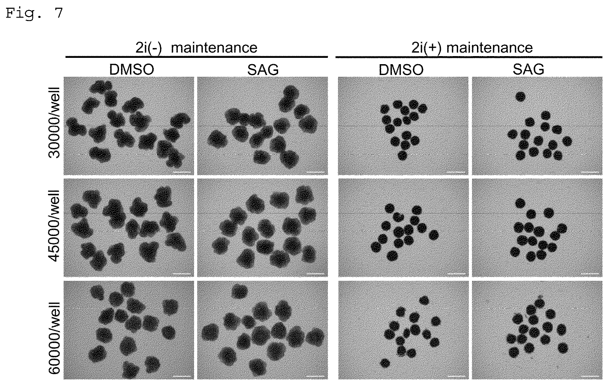

Fig. 7 shows brightfield images of the cell masses at Day 13. The cell mass morphology showed that no differentiation occurred when an undifferentiated maintenance medium was used instead of the intermediate medium.

### Test Example 5. Suprachiasmatic nucleus organoid production 5

Human iPS cells (201 B7) that had been cultured in adhesion in a feeder-free manner in an undifferentiated maintenance medium (composition: AK02N, Y27632 (ROCK inhibitor) 1 µM) were seeded into a 96-well plate (U-bottom well) at 10000 or 30000 cells/well and cultured in a medium (medium A) containing the undifferentiated maintenance medium and 1 µM of SAG. On the following day, the entire medium was transferred to a dish containing a neural differentiation medium (approximately equal amount, containing SAG and Y27632). On the next day, half of the medium was replaced with a neural differentiation medium (Y27632 and 1 uM of SAG). On the following day, half of the medium was replaced with a neural differentiation medium (Y27632 and 1 uM of SAG). On the day after that, the entire medium was replaced with a neural differentiation medium (Y27632 and 1 uM of SAG) (Day 0).

On Day 11, the cell masses were collected, washed once with a neural differentiation medium (expansion medium) (composition: DMEM/F12 (Gibco 10565), 10% KSR), and then seeded into an EZSphere to start suspension culture in the neural differentiation medium (expansion medium). Culturing was performed in a multi-gas incubator with 40% O₂ and 5% CO₂, and continued until Day 23. The medium was replaced every other day.

On Day 23, the medium was changed to a neural differentiation medium (maturation medium) (composition: DMEM/F12 (Gibco 10565), N2 supplements 1×, B27 (with Vitamin A) 1×), and the cells were continuously cultured in suspension. Culturing continued in the multi-gas incubator with 40% O₂ and 5% CO₂. The medium was replaced every other day.

The organoids collected on Day 26 were immunostained for suprachiasmatic nucleus markers, and the expression of SCN markers was confirmed.

## Claims

1. A method for producing a suprachiasmatic nucleus organoid, comprising:
(1) a step of culturing pluripotent stem cells in an intermediate medium between an undifferentiated maintenance medium and a neural differentiation medium;
(2) a step of culturing cells obtained in step (1) in the neural differentiation medium; and
(3) a step of performing an operation for cell mass formation prior to the start of step (2),
wherein
(A) the number of raw material cells seeded to form one cell mass is 6000 or greater, and
(B) when the start of step (2) is defined as Day 0, the medium contains a sonic hedgehog signaling pathway agonist prior to Day 3.

2. The production method according to claim 1, wherein the intermediate medium in step (1) is a medium in which the concentration of an undifferentiated maintenance factor is lower than that in the undifferentiated maintenance medium.

3. The production method according to claim 2, wherein the undifferentiated maintenance factor comprises an FGF-MAPK signal inhibitor and/or a Wnt signal activator.

4. The production method according to claim 1, wherein the number of the raw material cells in step (3) is 9000 or greater.

5. The production method according to claim 1, wherein the operation in step (3) comprises seeding cells into culture wells and culturing the cells in suspension.

6. The production method according to claim 1, wherein culturing in the medium containing the sonic hedgehog signaling pathway agonist continues until a day between Day 4 and Day 15.

7. The production method according to claim 1, wherein culturing in step (2) continues until a day between Day 4 and Day 15.

8. The production method according to claim 1, wherein the concentration of the sonic hedgehog signaling pathway agonist in the medium is 0.001 µM or more.

9. The production method according to claim 1, wherein the intermediate medium in step (1) is a medium in which the concentration of an undifferentiated maintenance factor is lower than that in the undifferentiated maintenance medium,
the undifferentiated maintenance factor comprises an FGF-MAPK signal inhibitor and/or a Wnt signal activator,
the number of the raw material cells in step (3) is 9000 or greater,
the operation in step (3) comprises seeding cells into culture wells and culturing the cells in suspension,
culturing in the medium containing the sonic hedgehog signaling pathway agonist continues until a day between Day 4 and Day 15,
culturing in step (2) continues until a day between Day 4 and Day 15, and
the concentration of the sonic hedgehog signaling pathway agonist in the medium is 0.001 µM or more.

10. The production method according to claim 1, further comprising (4) a step of culturing cell masses obtained in step (2) in the neural differentiation medium.

11. A suprachiasmatic nucleus organoid, wherein the proportion of suprachiasmatic nucleus cells is 1% or more.

12. The suprachiasmatic nucleus organoid according to claim 11, wherein the proportion of suprachiasmatic nucleus cells is 10% or more.

13. A suprachiasmatic nucleus organoid obtainable by the production method according to any one of claims 1 to 10.

14. A method for screening for a circadian rhythm regulator, comprising:
(X) a step of contacting the suprachiasmatic nucleus organoid according to claim 11 or 12 with a test substance; and
(Y) a step of evaluating the circadian rhythm in the suprachiasmatic nucleus organoid.

15. A circadian rhythm regulator having an action to change the circadian rhythm in the suprachiasmatic nucleus organoid according to claim 11 or 12.
